# EUROPEAN PATENT SPECIFICATION

(11) **EP 2 376 919 B1**
(45) Date of publication and mention of the grant of the patent: **29.04.2015**
(21) Application number: 09836707.1
(22) Date of filing: 08.12.2009
(51) Int. Cl.: G01N 33/53, G01N 33/74

(54) **COMBINED NATRIURETIC PEPTIDE ASSAYS**
KOMBINIERTE NATRIURETISCHE PEPTIDTESTS
DOSAGES DE PEPTIDES NATRIURÉTIQUES COMBINÉS

(30) Priority: 08.12.2008 US 120795 P
(43) Date of publication of application: 19.10.2011
(60) Divisional application: 15159206.0
(73) Proprietor: Alere San Diego, Inc., San Diego, CA 92121 (US)
(72) Inventor: LEE, Seok-Won, San Diego, CA 92130-5959 (US)
(74) Representative: Hoffmann Eitle
(86) International application number: PCT/US2009/067134
(87) International publication number: WO 2010/077654

(56) References cited:
- US-A1- 2004 176 914
- US-A1- 2008 160 540
- VICKERY SUSAN ET AL: "B-type natriuretic peptide (BNP) and amino-terminal proBNP in patients with CKD: relationship to renal function and left ventricular hypertrophy.", AMERICAN JOURNAL OF KIDNEY DISEASES : THE OFFICIAL JOURNAL OF THE NATIONAL KIDNEY FOUNDATION OCT 2005 LNKD- PUBMED:16183415, vol. 46, no. 4, October 2005 (2005-10), pages 610-620, XP002672362, ISSN: 1523-6838
- KEMPERMAN HANS ET AL: "B-type natriuretic peptide (BNP) and N-terminal proBNP in patients with end-stage heart failure supported by a left ventricular assist device.", CLINICAL CHEMISTRY SEP 2004 LNKD- PUBMED:15331503, vol. 50, no. 9, September 2004 (2004-09), pages 1670-1672, XP002672363, ISSN: 0009-9147
- LUCHNER ANDREAS ET AL: "Effect of compensated renal dysfunction on approved heart failure markers: direct comparison of brain natriuretic peptide (BNP) and N-terminal pro-BNP.", HYPERTENSION JUL 2005 LNKD- PUBMED:15939804, vol. 46, no. 1, July 2005 (2005-07), pages 118-123, XP002672364, ISSN: 1524-4563
- MADSEN L H ET AL: "N-terminal pro brain natriuretic peptide predicts mortality in patients with end-stage renal disease in hemodialysis.", KIDNEY INTERNATIONAL MAR 2007 LNKD- PUBMED:17299526, vol. 71, no. 6, March 2007 (2007-03), pages 548-554, XP002672365, ISSN: 0085-2538
- PHUA ET AL.: 'B-type natriuretic peptide: Issues for the intensivist and pulmonologist.' CRIT CARE MED vol. 33, 2005, pages 2094 - 2103, XP008143828
- PANTEGHINI ET AL.: 'Cardiac Natriuretic Hormones as Markers of Cardiovascular Disease: Methodological Aspects.' NATRIURETIC PEPTIDES. 2006, MILAN, pages 65 - 89, XP008143827
- HUNT ET AL.: 'The Amino Terminal Portion of Pro-Brain Natriuretic Peptide (pro-BNP) Circulates in Human Plasma.' BIOCHEMICAL AND BIOPHYSICAL RESEARCH COMMUNICATIONS vol. 214, no. 3, 25 September 1995, pages 1175 - 1183, XP000907386

## Description

### FIELD OF THE INVENTION

The present invention relates to use of natriuretic peptides as diagnostic and prognostic markers.

### BACKGROUND OF THE INVENTION

The following discussion of the background of the invention is merely provided to aid the reader in understanding the invention and is not admitted to describe or constitute prior art to the present invention.

Natriuretic peptides are a group of naturally occurring substances that act in the body to oppose the activity of the renin-angiotensin system. In recent years, natriuretic peptide measurement has dramatically changed the diagnosis and management of cardiac diseases, including heart failure and the acute coronary syndromes. In particular, B-type natriuretic peptide (BNP, human precursor Swiss-Prot P16860), and various related polypeptides arising from the common precursor proBNP, have been used to diagnose heart failure, determine its severity, and estimate prognosis.

There are three major natriuretic peptides: atrial natriuretic peptide (ANP), B-type natriuretic peptide (BNP), and C-type natriuretic peptide (CNP). Mature human B-type natriuretic peptide (BNP) (also called brain-type natriuretic peptide) is a 32 amino acid, 4 kDa peptide that is involved in the natriuresis system to regulate blood pressure and fluid balance. Bonow, R.O., Circulation 93:1946-1950, 1996. The precursor to BNP is synthesized as a 108-amino acid molecule, referred to as "pro-BNP" that is proteolytically processed into a 76-amino acid N-terminal peptide (amino acids 1-76), referred to as "NT-proBNP" and the 32-amino acid mature hormone, referred to as BNP or BNP 32 (amino acids 77-108). It has been suggested that each of these species - NT pro-BNP, BNP-32, and the pre-pro-BNP - together with various fragments thereof, can circulate in human plasma. *See, e.g.,* Tateyama et al., Biochem. Biophys. Res. Commun. 185:760-7, 1992; Hunt et al., Biochem. Biophys. Res. Commun. 214:1175-83, 1995); WO04/094459; and WO04094460, each of which is hereby incorporated in its entirety. In addition, the first two residues of BNP1-108 and BNP77-108 are removed by proteolysis, generating from proBNP the molecule BNP₃₋₁₀₈; from NT-proBNP the molecule BNP₃₋₇₆, and from BNP the molecule BNP₇₉₋₁₀₈. Assays and antibodies may be generated that are specific for the cleaved forms; for example, an assay that can detect BNP₃₋₁₀₈ but not proBNP (BNP₁₋₁₀₈); an assay that can detect BNP₃₋₇₆ but not NT-proBNP (BNP₁₋₇₆), and an assay that can detect BNP79-108 but not BNP₇₇₋₁₀₈.

The sequence of the human 108 amino acid BNP precursor pro-BNP (BNP₁₋₁₀₈) is as follows, with mature BNP (BNP₇₇₋₁₀₈) underlined:

HPLGSPGSAS DLETSGLQEQ RNHLQGKLSE LQVEQTSLEP LQESPRPTGV 50
WKSREVATEG IRGHRKMVLY TLRAPRSPKM VQGSGCFGRK MDRISSSSGL 100
GCKVLRRH 108
(SEQ ID NO: 1).

BNP₁₋₁₀₈ is synthesized as a larger precursor pre-pro-BNP having the following sequence (with the "pre" sequence shown in bold):

MDPQTAPSRA LLLLLFLHLA FLGGRSHPLG SPGSASDLET SGLQEQRNHL 50
QGKLSELQVE QTSLEPLQES PRPTGVWKSR EVATEGIRGH RKMVLYTLRA 100
PRSPKMVQGS GCFGRKMDRI SSSSGLGCKV LRRH 134

(SEQ ID NO: 2).

Alterations in natriuretic peptide levels may be seen in patients with renal dysfunction. BNP is released in response to ventricular stretch, and causes vasorelaxation, inhibition of aldosterone secretion in the adrenal cortex, and inhibition of renin secretion in the kidney. The term "cardiorenal syndrome" is used to refer to the physiologic relationship between the heart and kidney that manifests as a tight coordination between renal and cardiac functions in subjects suffering from heart failure. While the syndrome is poorly understood, a feedback loop amongst neurohormonal systems (and, in particular, the natriuretic peptides), inflammatory responses, and structurally and functionally impaired organs has been implicated, creating a cycle of worsening cardiac and renal functions. A recent discussion of cardiorenal syndrome may be found in Francis, "Acute decompensated heart failure: The cardiorenal syndrome," Clev. Clinic J. Med. 73(S2): S8-S13, 2006.

In addition, natriuretic peptide levels may also be altered in venous thromboembolic disease ("VTED"). VTED represents a spectrum of conditions that includes deep venous thrombosis (DVT) and pulmonary embolism (PE). The estimated annual incidence of VTED is 117 cases per 100,000 persons. The incidence rises markedly in persons 60 years and older and may be as high as 900 cases per 100,000 by the age of 85 years. Silverstein et al., Arch. Intern. Med. 158: 585-93, 1998. Risk factors for VTED include increasing age, prolonged immobility, surgery, trauma, malignancy, pregnancy, estrogenic medications (e.g., oral contraceptive pills, hormone therapy, tamoxifen), congestive heart failure, hyperhomocystinemia, diseases that alter blood viscosity (e.g., polycythemia, sickle cell disease, multiple myeloma), and inherited thrombophilias. About 75 percent of patients with VTED have at least one established risk factor. Heit et al., Arch. Intern. Med. 162: 1245-48, 2002.

### SUMMARY OF THE INVENTION

The present invention relates to the use of natriuretic peptide assays to distinguish between renal dysfunction and/or heart failure from pulmonary embolism in subjects. Subjects with these conditions often present with similar overt symptoms, such as dyspnea, chest pain, cough, edema, etc. By combining the results from two or more assays that detect different populations of BNP-related polypeptides, an initial differential diagnosis which includes both renal dysfunction and pulmonary embolism may be refined.

In a first aspect of the present invention, at least two immunoassays selected from the group consisting of an immunoassay configured to detect BNP₇₉₋₁₀₈, and optionally also detecting BNP itself (that is, BNP₇₇₋₁₀₈); an immunoassay configured to detect BNP₃₋₇₆, and optionally also detecting BNP₁₋₇₆, but not to detect BNP; and an immunoassay configured to detect BNP₃₋₁₀₈, and optionally also detecting BNP₁₋₁₀₈, but not to detect BNP, are performed on a sample from a subject. The results of these immunoassays are combined computationally, and the computational result is then used to assign a diagnosis of the presence or absence of renal dysfunction, and/or the presence or absence of pulmonary embolism, and/or the presence or absence of heart failure, and/or to distinguish renal dysfunction from heart failure and/or pulmonary embolism in the subject.

As is described in detail hereinafter, an assay is "configured to detect" a particular marker of interest if that assay generates a detectable signal indicative of the presence or amount of a physiologically relevant concentration of that marker. Such an assay may, but need not, specifically detect a particular natriuretic peptide. Because an antibody epitope is on the order of 8 amino acids, an immunoassay will detect other polypeptides (*e.g.,* related markers) so long as the other polypeptides contain the epitope(s) necessary to bind to the antibody used in the assay. That is, an assay that detects BNP₇₉₋₁₀₈ may also detect BNP, proBNP, and BNP₃₋₁₀₈,

In the exemplary embodiments, an assay to detect BNP₇₉₋₁₀₈ is configured as a sandwich immunoassay using two antibodies that bind to epitopes contained within the BNP₇₉₋₁₀₈ sequence. One such assay utilizes one antibody that is specific for the loss of residues 77 and 78 from the BNP molecule, and a second antibody that binds to both BNP and BNP₇₉₋₁₀₈; such an assay will not detect proBNP, BNP₃₋₁₀₈, NT-proBNP, or BNP₃₋₇₆, and is referred to herein as a "BNP₇₉₋₁₀₈ assay." A second such assay utilizes two antibodies, each of which bind both BNP and BNP₇₉₋₁₀₈; such an assay will also detect proBNP and BNP₃₋₁₀₈, and is referred to herein as a "BNP assay."

In other exemplary embodiments, an assay to detects BNP₃₋₇₆, but not BNP is configured as a sandwich immunoassay using two antibodies that bind to epitopes contained within the BNP₃₋₇₆ sequence. One such assay utilizes one antibody that is specific for the loss of residues 1 and 2 from the NT-proBNP molecule, and a second antibody that binds to both NT-proBNP and BNP₋₇₆; such an assay will not detect proBNP, BNP,BNP₇₉₋₁₀₈, or NT-proBNP, and is referred to herein as a "BNP₃₋₇₆ assay." A second such assay utilizes two antibodies, each of which bind both NT-proBNP and BNP₃₋₇₆; such an assay will also detect proBNP and BNP₃₋₁₀₈, and is referred to herein as a "NT-proBNP assay."

In other exemplary embodiments described hereinafter, an assay to detect BNP₃₋₁₀₈, but not BNP is configured as a sandwich immunoassay using two antibodies that bind to epitopes contained within the BNP₃₋₁₀₈ sequence, at least one of which does not bind to BNP. One such assay utilizes one antibody that is specific for the loss of residues 1 and 2 from the proBNP molecule, and a second antibody that binds to both proBNP and BNP₃₋₁₀₉; such an assay will not detect proBNP, BNP,BNP₇₉₋₁₀₈, or NT-proBNP, and is referred to herein as a "BNP₃₋₁₀₈ assay." A second such assay utilizes two antibodies, each of which bind both proBNP and BNP₋₁₀₈; such an assay will not detect BNP, and is referred to herein as a "proBNP assay."

The assays are selected such that one can computationally derive at least the following concentrations from the assay results: a concentration that is substantially indicative of a concentration that is substantially indicative of proBNP or BNP₃₋₁₀₈ (each of which is referred to herein as a "proBNP concentration") and a concentration that is substantially indicative of NT-proBNP or BNP₃₋₇₆ (each of which is referred to herein as an "NT-proBNP concentration").

By a concentration that is "substantially indicative" of BNP or BNP₇₉₋₁₀₈ is meant that the concentration has been deconvoluted by removing contributions of proBNP or BNP₃₋₁₀₈ and NT-proBNP or BNP₃₋₇₆. In one example, a "BNP₇₉₋₁₀₈ assay" result may be used directly, as proBNP, BNP₃₋₁₀₈, NT-proBNP and BNP₃₋₇₆ do not contribute to such an assay result. In another example, a "BNP assay" result may be used by subtracting the results of a "proBNP assay," as this will correct for the proBNP contribution to the BNP assay result.

Likewise, by a concentration that is "substantially indicative" of proBNP or BNP₃₋₁₀₈ is meant that the concentration has been deconvoluted by removing contributions of BNP or BNP₇₉₋₁₀₈ andNT-proBNP or BNP₃₋₇₆. And by a concentration that is "substantially indicative" of NT-proBNP or BNP₃₋₇₆ is meant that the concentration has been deconvoluted by removing contributions of proBNP or BNP₃₋₁₀₈ and BNP or BNP₇₉₋₁₀₈.

These concentrations are then combined to determine a "combined natriuretic peptide result" which is a function of proBNP concentration and NT-proBNP concentration. In preferred embodiments, such a function can be a a proBNP/NT-proBNP ratio or an NT-proBNP/proBNP ratio. The ratio is used to diagnose the presence or absence of renal dysfunction.

The combined natriuretic peptide result may be analyzed in a number of fashions well known to those of skill in the art. For example, the result obtained may be compared to a "normal" value, or a value indicating a particular disease or outcome. A particular diagnosis/prognosis may depend upon the comparison of each assay result to such a value, which may be referred to as a diagnostic or prognostic "threshold." The sensitivity and specificity of a diagnostic and/or prognostic test depends on more than just the analytical "quality" of the testthey also depend on the definition of what constitutes an abnormal result. In practice, Receiver Operating Characteristic curves, or "ROC" curves, are typically calculated by plotting the value of a variable versus its relative frequency in "normal" and "disease" populations. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. These methods are well known in the art. *See, e.g.,* Hanley et al., Radiology 143: 29-36 (1982).

A positive likelihood ratio, negative likelihood ratio, odds ratio, or hazard ratio may be used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, a threshold is selected to distinguish pulmonary embolism and/or heart failure from renal dysfunction in a subject with a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, a threshold is selected to distinguish heart failure and/or pulmonary embolism from renal dysfunction in a subject with an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (*e.g.,* death) is equal in both the "diseased" and "control" groups; a value greater than 1 indicates that the risk is greater in the diseased group; and a value less than 1 indicates that the risk is greater in the control group. In certain preferred embodiments, a threshold is selected to distinguish heart failure and/or pulmonary embolism from renal dysfunction in a subject with a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement.

The skilled artisan will understand that associating a diagnostic or prognostic indicator, with a diagnosis or with a prognostic risk of a future clinical outcome is a statistical analysis. For example, a marker level of greater than X may signal that a patient is more likely to suffer from an adverse outcome than patients with a level less than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, *e.g*., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

### BRIEF DESCRIPTION OF THE FIGURES

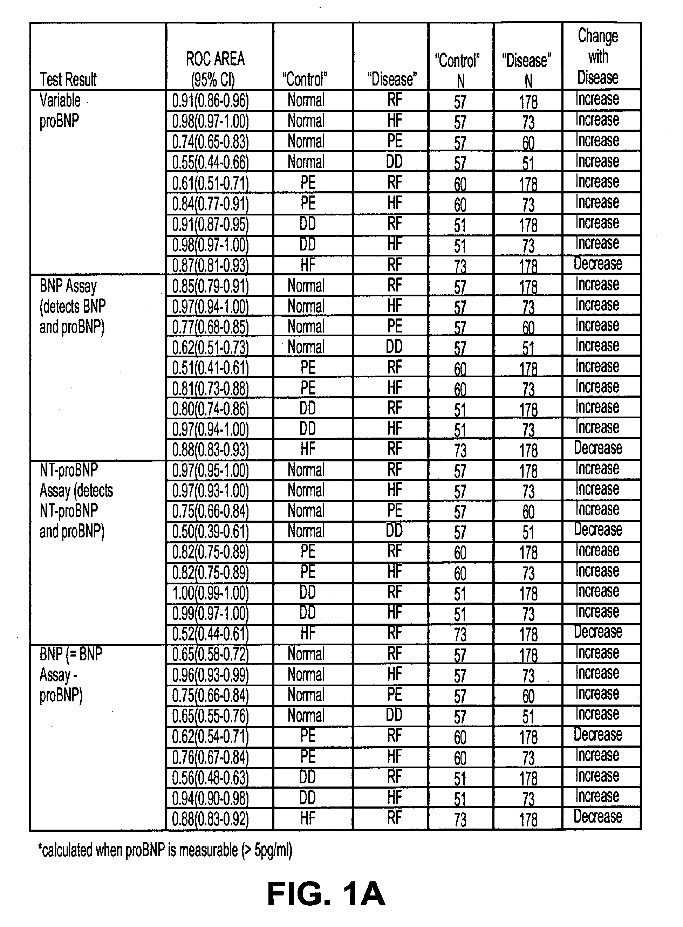

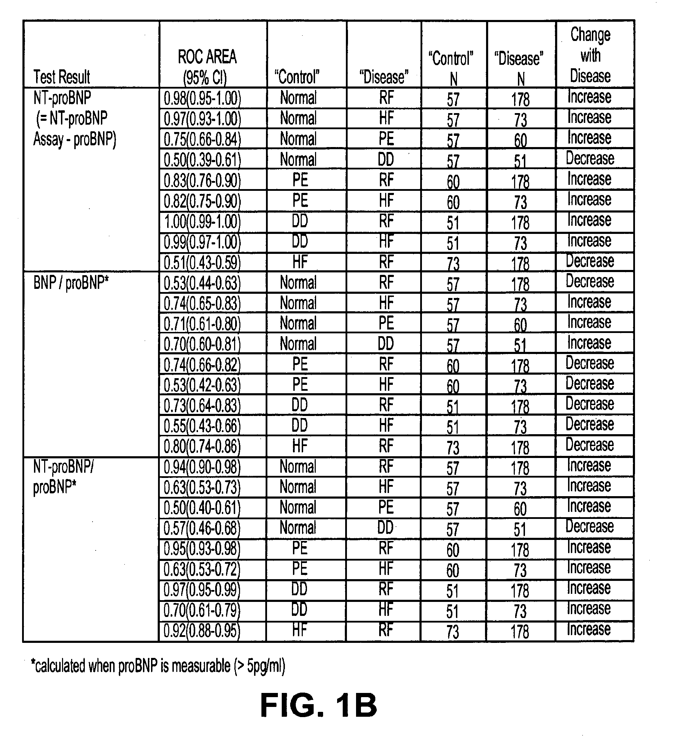

Fig. 1A and 1B depict Receiver Operator Characteristic data calculated from individual Natriuretic peptide (proBNP, BNP, and NT-proBNP) assays, and combined Natriuretic peptide assays in normal and diseased populations.

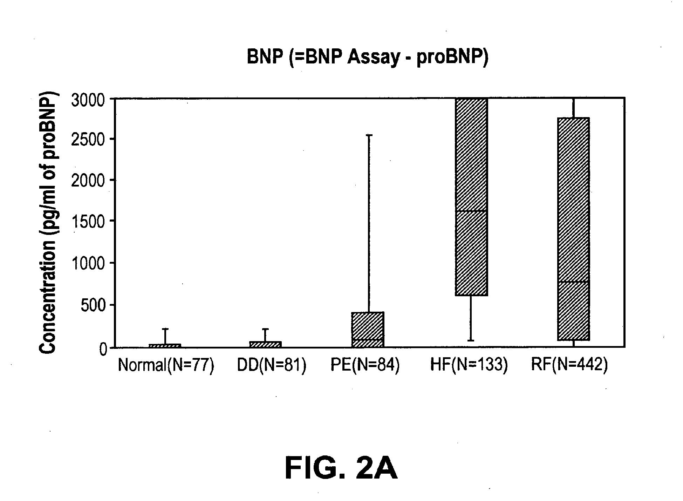

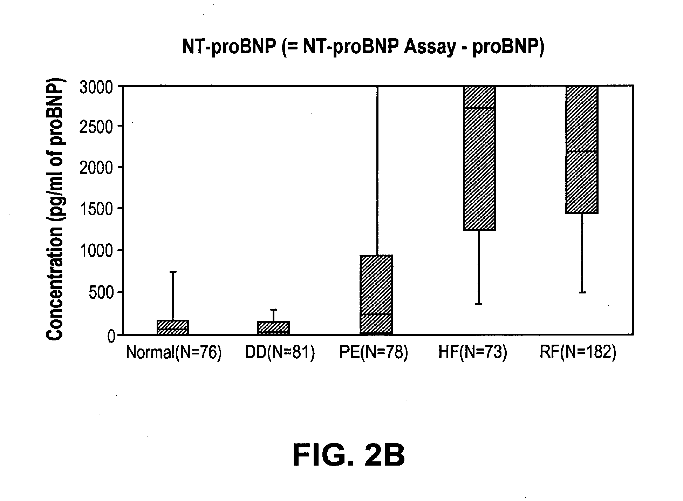

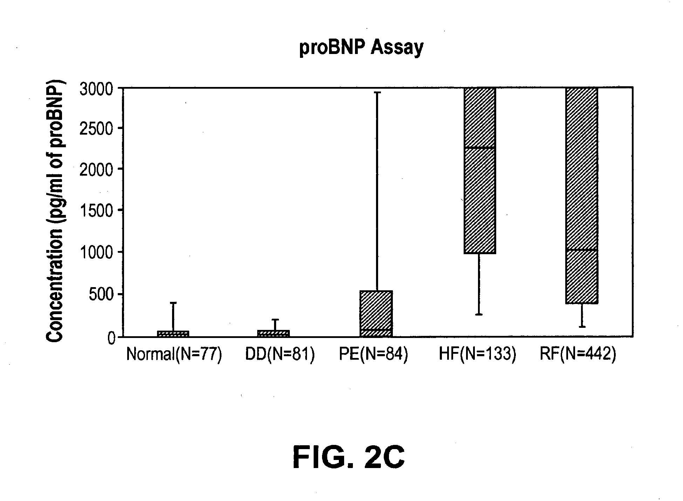

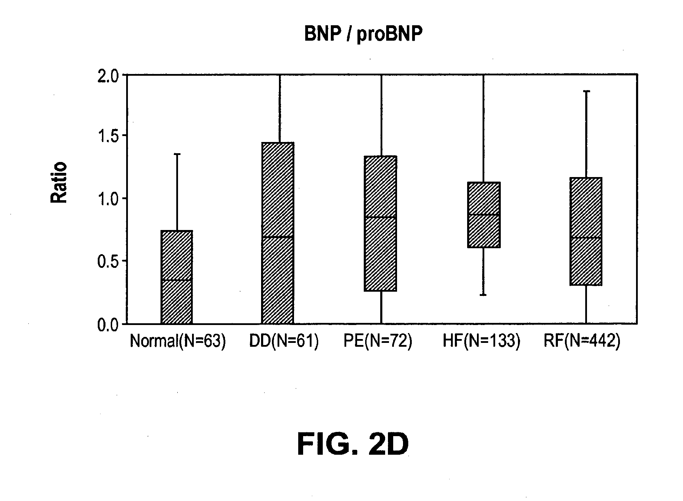

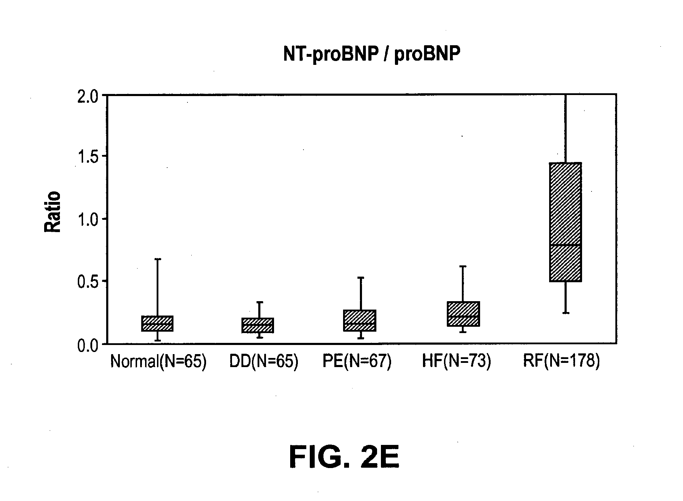

Fig. 2A-2E depict box-and-whisker plots obtained from individual Natriuretic peptide (proBNP, BNP, and NT-proBNP) assays, and combined Natriuretic peptide assays in normal and diseased populations. In Fig. 2A, 2B, and 2C, numbers on the Y axis are concentration in pg/ml of proBNP. In Fig. 2D and 2E, numbers on the Y axis indicate the ratio of BNP/proBNP (Fig. 2D) and NT-proBNP/proBNP (Fig. 2E).

### DETAILED DESCRIPTION OF THE INVENTION

As described herein, an assay that detects BNP₇₉₋₁₀₈, for example a sandwich immunoassay using two antibodies that bind to epitopes contained within the BNP₇₉₋₁₀₈ sequence, may also detect other markers related to BNP₇₉₋₁₀₈. An antibody epitope is on the order of 8 amino acids; thus, an immunoassay will detect other polypeptides (e.g., related markers) so long as the other polypeptides contain the epitope(s) necessary to bind to the antibody used in the assay. Thus, such an assay may also, but need not, detect BNP, proBNP, BNP₃₋₁₀₈, and fragments of such polypeptides that provide the necessary antibody binding characteristics. For example, an assay that utilizes one antibody that is specific for the loss of residues 77 and 78 from the BNP molecule, and a second antibody that binds to both BNP and BNP₇₉₋₁₀₈; will not detect proBNP, BNP₃₋₁₀₈, NT-proBNP, or BNP₃₋₇₆. An assay that utilizes two antibodies, each of which bind both BNP and BNP₇₉₋₁₀₈ will also detect proBNP and BNP₃₋₁₀₈. Each is "an assay that detects BNP₇₉₋₁₀₈."

Likewise, an assay that detects BNP₃₋₇₆ but not BNP, for example a sandwich immunoassay using two antibodies that bind to epitopes contained within the BNP₃₋₇₆ sequence, may also, but need not, detect other markers related to BNP₃₋₇₆ that provide the necessary antibody binding characteristics. In one example, an assay utilizes one antibody that is specific for the loss of residues 1 and 2 from the NT-proBNP molecule, and a second antibody that binds to both NT-proBNP and BNP₃₋₇₆; such an assay will not detect proBNP, BNP, BNP₇₉₋₁₀₈, or NT-proBNP. In another example, an assay utilizes two antibodies, each of which bind both NT-proBNP and BNP₃₋₇₆; such an assay will also detect proBNP and BNP₃₋₁₀₈. Each is "an assay that detects BNP₃₋₇₆ but not BNP."

And similarly, an assay that detects BNP₃₋₁₀₈ but not BNP , for example a sandwich immunoassay using two antibodies that bind to epitopes contained within the BNP₃₋₁₀₈ sequence, at least one of which does not bind to BNP, may also, but need not, detect other markers related to BNP₃₋₁₀₈ that provide the necessary antibody binding characteristics. One such assay utilizes one antibody that is specific for the loss of residues 1 and 2 from the proBNP molecule, and a second antibody that binds to both proBNP and BNP₃₋₁₀₈; such an assay will not detect proBNP, BNP, BNP₇₉₋₁₀₈, or NT-proBNP. A second such assay utilizes two antibodies, each of which bind both proBNP and BNP₃₋₁₀₈; such an assay will not detect BNP or BNP₇₉₋₁₀₈. Each is "an assay that detects BNP₃₋₁₀₈ but not BNP."

Using these assays, a concentration that is "substantially indicative" of BNP or BNP₇₉₋₁₀₈ may be calculated. In one example, a "BNP₇₉₋₁₀₈ assay" result may be used directly, as proBNP, BNP₃₋₁₀₈, NT-proBNP and BNP₃₋₇₆ do not contribute to the assay result. In another example, a "BNP assay" result which includes a contribution from proBNP and BNP₃₋₁₀₈ may be used by subtracting the results of an assay that detects BNP₃₋₁₀₈ but not BNP, as this will correct for the proBNP-related contribution to the BNP assay result, thus "deconvoluting" the BNP assay. As defined herein, an assay result that is "substantially indicative" of BNP or BNP₇₉₋₁₀₈ in this manner is referred to as a "BNP concentration."

Likewise, a concentration that is "substantially indicative" of proBNP or BNP₃₋₁₀₈ may be calculated. In one example, a BNP₃₋₁₀₈ assay may be used directly, as BNP, BNP₇₉₋₁₀₈, NT-proBNP, and BNP₃₋₇₆ do not contribute to the assay result. In another example, a "BNP₁₋₁₀₈ assay" result which includes a contribution from NT-proBNP, and BNP₃₋₇₆ may be used by subtracting the results of an assay that detects BNP₃₋₇₆ but not BNP, as this will correct for the NTproBNP-related contribution to the BNP₁₋₁₀₈ assay result. As defined herein, an assay result that is "substantially indicative" of proBNP or BNP₃₋₁₀₈ in this manner is referred to as a "proBNP concentration."

And a concentration that is "substantially indicative" of NT-proBNP or BNP₃₋₇₆ may be measured directly, or by removing contributions of proBNP or BNP₃₋₁₀₈ and BNP or BNP₇₉₋₁₀₈ from an assay that detects each of the Natriuretic peptide forms. As defined herein, an assay result that is "substantially indicative" of NT-proBNP or BNP₃₋₇₆ in this manner is referred to as an "NTproBNP concentration."

These concentrations are then combined to determine a "combined natriuretic peptide result" which is a function of a proBNP concentration and an NT-proBNP concentration. In preferred embodiments, such a function can be a proBNP/NT-proBNP ratio or an NT-proBNP/proBNP ratio. The combined natriuretic peptide result is used as a diagnostic or prognostic marker in accordance with standard methodology well known in the art.

### Definitions

The term "marker" as used herein refers to proteins, polypeptides, phospholipids, or small molecules to be used as targets for screening test samples obtained from subjects. "Proteins or polypeptides" used as markers in the present invention are contemplated to include any fragments thereof, in particular, immunologically detectable fragments. A "marker" may also be derived from individual marker measurements, for example as in the case of a "combined natriuretic peptide result."

The term "related marker" as used herein refers to one or more fragments of a particular marker or its biosynthetic parent that may be detected as a surrogate for the marker itself or as independent markers. For example, human BNP is derived by proteolysis of a 108 amino acid precursor molecule, referred to hereinafter as BNP₁₋₁₀₈. Mature BNP, or "the BNP natriuretic peptide," or "BNP-32" is a 32 amino acid molecule representing amino acids 77-108 of this precursor, which may be referred to as BNP₇₇₋₁₀₈. The remaining residues 1-76 are referred to hereinafter as BNP₁₋₇₆. Many of the markers described herein are synthesized as larger precursor molecules, which are then processed to provide mature marker; and/or are present in circulation in the form of fragments of the marker. Thus, "related markers" to each of the markers described herein may be identified and used in an analogous fashion to that described above for BNP. Additionally, related markers may be the result of covalent modification of the parent marker, for example by oxidation of methionine residues, ubiquitination, *etc.*

The term "test sample" as used herein refers to a sample of bodily fluid obtained for the purpose of diagnosis, prognosis, or evaluation of a subject of interest, such as a patient. In certain embodiments, such a sample may be obtained for the purpose of determining the outcome of an ongoing condition or the effect of a treatment regimen on a condition. Preferred test samples include blood, serum, plasma, cerebrospinal fluid, urine, saliva, sputum, and pleural effusions. In addition, one of skill in the art would realize that some test samples would be more readily analyzed following a fractionation or purification procedure, for example, separation of whole blood into serum or plasma components.

As used herein, a "plurality" refers to at least two. Preferably, a plurality refers to at least 3, more preferably at least 5, even more preferably at least 10, even more preferably at least 15, and most preferably at least 20. In particularly preferred embodiments, a plurality is a large number, i.e., at least 100.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are "patients," i.e., living humans that are receiving medical care. This includes persons with no defined illness who are being investigated for signs of pathology. In the case of the present invention, preferred subjects to be evaluated for the presence or absence of subclinical atherosclerosis are individuals not exhibiting clinical symptoms, most preferably chest pain or ECG changes, indicative of stable angina, unstable angina, and/or myocardial infarction. Preferred subjects to be evaluated prognostically are individuals not exhibiting clinical symptoms, most preferably chest pain or ECG changes, indicative of stable angina, unstable angina, and/or myocardial infarction, but who have been assigned a diagnosis of subclinical atherosclerosis.

The term "diagnosis" as used herein refers to methods by which the skilled artisan can estimate and/or determine whether or not a patient is suffering from a given disease or condition. The skilled artisan often makes a diagnosis on the basis of one or more diagnostic indicators, *i.e.,* a marker, the presence, absence, amount, or change in amount of which is indicative of the presence, severity, or absence of the condition.

Similarly, a "prognosis" refers to assignment of a probability that a given course or outcome will occur. This is often determined by examining one or more "prognostic indicators." These are markers, the presence or amount of which in a patient (or a sample obtained from the patient) signal a probability that a given course or outcome will occur. For example, when one or more prognostic indicators reach a sufficiently high level in samples obtained from such patients, the level may signal that the patient is at an increased probability for experiencing a future stroke in comparison to a similar patient exhibiting a lower marker level. A level or a change in level of a prognostic indicator, which in turn is associated with an increased probability of morbidity or death, is referred to as being "associated with an increased predisposition to an adverse outcome" in a patient.

The term "correlating," as used herein in reference to the use of diagnostic and prognostic markers, refers to comparing the presence or amount of the marker(s) in a patient to its presence or amount in persons known to suffer from, or known to be at risk of, a given condition; or in persons known to be free of a given condition. As discussed above, a marker level in a patient sample can be compared to a level known to be associated with a specific diagnosis. The sample's marker level is said to have been correlated with a diagnosis; that is, the skilled artisan can use the marker level to determine whether the patient suffers from a specific type diagnosis, and respond accordingly. Alternatively, the sample's marker level can be compared to a marker level known to be associated with a good outcome (*e.g*., the absence of disease, *etc.*)*.*

The phrase "determining the diagnosis" as used herein refers to methods by which the skilled artisan can determine the presence or absence of a particular disease in a patient. The term "diagnosis" does not refer to the ability to determine the presence or absence of a particular disease with 100% accuracy, or even that a given course or outcome is more likely to occur than not. Instead, the skilled artisan will understand that the term "diagnosis" refers to an increased probability that a certain disease is present in the subject. In preferred embodiments, a diagnosis indicates about a 5% increased chance that a disease is present, about a 10% chance, about a 15% chance, about a 20% chance, about a 25% chance, about a 30% chance, about a 40% chance, about a 50% chance, about a 60% chance, about a 75% chance, about a 90% chance, and about a 95% chance. The term "about" in this context refers to +/- 2%.

Similarly, the phrase "determining the prognosis" as used herein refers to methods by which the skilled artisan can determine the likelihood of one or more future clinical outcomes for a patient. The skilled artisan will understand that the term "prognosis" refers to an increased probability that a certain clinical outcome will occur at a future date in the subject. In preferred embodiments, a prognosis indicates about a 5% increased chance of a certain clinical outcome compared to a "control" population, about a 10% chance, about a 15% chance, about a 20% chance, about a 25% chance, about a 30% chance, about a 40% chance, about a 50% chance, about a 60% chance, about a 75% chance, about a 90% chance, and about a 95% chance. The term "about" in this context refers to +/- 2%.

The term "renal dysfunction" as used herein refers to a subject that falls within or is at an increased risk within 48 hours of falling within the acute kidney injury classification scheme in Mehta et al., Crit. Care 11:R31 (doi:10.1186.cc5713), 2007, hereby incorporated by reference in its entirety:
"Stage I": increase in serum creatinine of more than or equal to 0.3 mg/dL (≥ 26.4 µmol/L) or increase to more than or equal to 150% (1.5-fold) from baseline OR urine output less than 0.5 mL/kg per hour for more than 6 hours;
"Stage II": increase in serum creatinine to more than 200% (> 2-fold) from baseline OR urine output less than 0.5 mL/kg per hour for more than 12 hours;
"Stage III": increase in serum creatinine to more than 300% (> 3-fold) from baseline OR urine output less than 0.3 mL/kg per hour for 24 hours or anuria for 12 hours.

The term "heart failure" as used herein refers to a subject that falls within the American College of Cardiology/American Heart Association heart failure classification scheme in Hunt et al., "ACC/AHA 2005 Guideline Update for the Diagnosis and Management of Chronic Heart Failure in the Adult," Circulation 112 (12): e154-235 (doi:10.1161/ CIRCULATIONAHA.105.167586),2005:
"Stage A": Patients at high risk for developing HF in the future but no functional or structural heart disorder;
"Stage B": a structural heart disorder but no symptoms at any stage;
"Stage C": previous or current symptoms of heart failure in the context of an underlying structural heart problem, but managed with medical treatment;
"Stage D": advanced disease requiring hospital-based support, a heart transplant or palliative care.

The term "pulmonary embolism" as used herein refers to a subject suffering from a blockage of the pulmonary artery or one of its branches, usually occurring when a venous thrombus (blood clot from a vein) becomes dislodged from its site of formation and embolizes to the arterial blood supply of one of the lungs.

The term "discrete" as used herein refers to areas of a surface that are non-contiguous. That is, two areas are discrete from one another if a border that is not part of either area completely surrounds each of the two areas.

The term "independently addressable" as used herein refers to discrete areas of a surface from which a specific signal may be obtained.

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 3.41:544-546), which consists of a VH domain; and (vi) an isolated complementarity determining region (CDR). Single chain antibodies are also included by reference in the term "antibody."

The term "specifically binds" is not intended to indicate that an antibody binds exclusively to its intended target. Rather, an antibody "specifically binds" if its affinity for its intended target is about 5-fold greater when compared to its affinity for a non-target molecule. Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Specific binding between an antibody or other binding agent and an antigen means a binding affinity of at least 10⁶ M⁻¹. Preferred antibodies bind with affinities of at least about 10⁷ M⁻¹, and preferably between about 10⁸ M⁻¹ to about 10⁹ M⁻¹, about 10⁹ M⁻¹ to about 10¹⁰ M⁻¹, or about 10¹⁰ M⁻¹ to about 10¹¹ M⁻¹.

Affinity is calculated as K_{d} =k_{off} /kₒₙ (k_{off} is the dissociation rate constant, kₒₙ is the association rate constant and K_{d} is the equilibrium constant. Affinity can be determined at equilibrium by measuring the fraction bound (r) of labeled ligand at various concentrations (c). The data are graphed using the Scatchard equation: r/c = K(n-r):
where
r = moles of bound ligand/mole of receptor at equilibrium;
c = free ligand concentration at equilibrium;
K = equilibrium association constant; and
n = number of ligand binding sites per receptor molecule
By graphical analysis, r/c is plotted on the Y-axis versus r on the X-axis thus producing a Scatchard plot. The affinity is the negative slope of the line. k_{off} can be determined by competing bound labeled ligand with unlabeled excess ligand (see, e.g., U.S. Pat No. 6,316,409). The affinity of a targeting agent for its target molecule is preferably at least about 1 x 10⁻⁶ moles/liter, is more preferably at least about 1 x 10⁻⁷ moles/liter, is even more preferably at least about 1 x 10⁻⁸ moles/liter, is yet even more preferably at least about 1 x 10⁻⁹ moles/liter, and is most preferably at least about 1 x 10⁻¹⁰ moles/liter. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

Measures of test accuracy may be obtained as described in Fischer et al., Intensive Care Med. 29: 1043-51, 2003, and used to determine the effectiveness of a given marker or panel of markers. These measures include sensitivity and specificity, predictive values, likelihood ratios, diagnostic odds ratios, and ROC curve areas. As discussed above, preferred tests and assays exhibit one or more of the following results on these various measures:
at least 75% sensitivity, combined with at least 75% specificity;

ROC curve area of at least 0.6, more preferably 0.7, still more preferably at least 0.8, even more preferably at least 0.9, and most preferably at least 0.95; and/or a positive likelihood ratio (calculated as sensitivity/(1-specificity)) of at least 5, more preferably at least 10, and most preferably at least 20, and a negative likelihood ratio (calculated as (1-sensitivity)/specificity) of less than or equal to 0.3, more preferably less than or equal to 0.2, and most preferably less than or equal to 0.1.

### Assay Measurement Strategies

Numerous methods and devices are well known to the skilled artisan for the detection and analysis of the markers of the instant invention. With regard to polypeptides or proteins in patient test samples, immunoassay devices and methods are often used*. See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, each of which is hereby incorporated by reference in its entirety, including all tables, figures and claims. These devices and methods can utilize labeled molecules in various sandwich, competitive, or non-competitive assay formats, to generate a signal that is related to the presence or amount of an analyte of interest. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman Access, Abbott AxSym, Roche ElecSys, Dade Behring Stratus systems are among the immunoassay analyzers that are capable of performing the immunoassays taught herein.

Preferably the markers are analyzed using an immunoassay, and most preferably sandwich immunoassay, although other methods are well known to those skilled in the art (for example, the measurement of marker RNA levels). The presence or amount of a marker is generally determined using antibodies specific for each marker and detecting specific binding. Any suitable immunoassay may be utilized, for example, enzyme-linked immunoassays (ELISA), radioimmunoassays (RIAs), competitive binding assays, and the like. Specific immunological binding of the antibody to the marker can be detected directly or indirectly. Direct labels include fluorescent or luminescent tags, metals, dyes, radionuclides, and the like, attached to the antibody. Indirect labels include various enzymes well known in the art, such as alkaline phosphatase, horseradish peroxidase and the like.

The use of immobilized antibodies specific for the markers is also contemplated by the present invention. The antibodies could be immobilized onto a variety of solid supports, such as magnetic or chromatographic matrix particles, the surface of an assay plate (such as microtiter wells), pieces of a solid substrate material or membrane (such as plastic, nylon, paper), and the like. An assay strip could be prepared by coating the antibody or a plurality of antibodies in an array on solid support. This strip could then be dipped into the test sample and then processed quickly through washes and detection steps to generate a measurable signal, such as a colored spot.

For separate or sequential assay of markers, suitable apparatuses include clinical laboratory analyzers such as the ElecSys (Roche), the AxSym (Abbott), the Access (Beckman), the ADVIA® CENTAUR® (Bayer) immunoassay systems, the NICHOLS ADVANTAGE® (Nichols Institute) immunoassay system, etc. Preferred apparatuses perform simultaneous assays of a plurality of markers using a single test device. Particularly useful physical formats comprise surfaces having a plurality of discrete, addressable locations for the detection of a plurality of different analytes. Such formats include protein microarrays, or "protein chips" (*see, e.g.,* Ng and Ilag, J. Cell Mol. Med. 6: 329-340 (2002)) and certain capillary devices (*see, e.g.,* U.S. Patent No. 6,019,944). In these embodiments, each discrete surface location may comprise antibodies to immobilize one or more analyte(s) (*e.g*., a marker) for detection at each location. Surfaces may alternatively comprise one or more discrete particles (*e.g*., microparticles or nanoparticles) immobilized at discrete locations of a surface, where the microparticles comprise antibodies to immobilize one analyte (*e.g.,* a marker) for detection.

Preferred assay devices of the present invention will comprise, for one or more assays, a first antibody conjugated to a solid phase and a second antibody conjugated to a signal development element. Such assay devices are configured to perform a sandwich immunoassay for one or more analytes. These assay devices will preferably further comprise a sample application zone, and a flow path from the sample application zone to a second device region comprising the first antibody conjugated to a solid phase.

Flow of a sample along the flow path may be driven passively (*e.g.,* by capillary, hydrostatic, or other forces that do not require further manipulation of the device once sample is applied), actively (*e.g.,* by application of force generated via mechanical pumps, electroosmotic pumps, centrifugal force, increased air pressure, *etc.),* or by a combination of active and passive driving forces. Most preferably, sample applied to the sample application zone will contact both a first antibody conjugated to a solid phase and a second antibody conjugated to a signal development element along the flow path (sandwich assay format). Additional elements, such as filters to separate plasma or serum from blood, mixing chambers, *etc.,* may be included as required by the artisan. Exemplary devices are described in Chapter 41, entitled "Near Patient Tests: Triage® Cardiac System," in The Immunoassay Handbook, 2nd ed., David Wild, ed., Nature Publishing Group, 2001, which is hereby incorporated by reference in its entirety.

The analysis of markers could be carried out in a variety of physical formats as well. For example, the use of microtiter plates or automation could be used to facilitate the processing of large numbers of test samples. Alternatively, single sample formats could be developed to facilitate immediate treatment and diagnosis in a timely fashion, for example, in ambulatory transport or emergency room settings.

The clinical sensitivity of an assay is defined as the percentage of those with the disease that the assay correctly predicts, and the specificity of an assay is defined as the percentage of those without the disease that the assay correctly predicts (Tietz Textbook of Clinical Chemistry, 2nd edition, Carl Burtis and Edward Ashwood eds., W.B. Saunders and Company, p. 496). Measures of test accuracy may be obtained as described in Fischer et al., Intensive Care Med. 29: 1043-51, 2003, and used to determine the effectiveness of a given marker or panel of markers. These measures include sensitivity and specificity, predictive values, likelihood ratios, diagnostic odds ratios, and ROC curve areas. As discussed above, preferred tests and assays exhibit one or more of the following results on these various measures:
the ability to distinguish pulmonary embolism from renal dysfunction in a subject with a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement;
the ability to distinguish pulmonary embolism from renal dysfunction in a subject with an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement;
the ability to distinguish pulmonary embolism from renal dysfunction in a subject with a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement.

### Selection of Antibodies

The generation and selection of antibodies may be accomplished several ways. For example, one way is to purify polypeptides of interest or to synthesize the polypeptides of interest using, *e.g.,* solid phase peptide synthesis methods well known in the art. *See, e.g.,* Guide to Protein Purification, Murray P. Deutcher, ed., Meth. Enzymol. Vol 182 (1990); Solid Phase Peptide Synthesis, Greg B. Fields ed., Meth. Enzymol. Vol 289 (1997); Kiso et al., Chem. Pharm. Bull. (Tokyo) 38: 1192-99, 1990; Mostafavi et al., Biomed. Pept. Proteins Nucleic Acids 1: 255-60, 1995; Fujiwara et al., Chem. Pharm. Bull. (Tokyo) 44: 1326-31, 1996. The selected polypeptides may then be injected, for example, into mice or rabbits, to generate polyclonal or monoclonal antibodies. One skilled in the art will recognize that many procedures are available for the production of antibodies, for example, as described in Antibodies, A Laboratory Manual, Ed Harlow and David Lane, Cold Spring Harbor Laboratory (1988), Cold Spring Harbor, N.Y. One skilled in the art will also appreciate that binding fragments or Fab fragments which mimic antibodies can also be prepared from genetic information by various procedures (Antibody Engineering: A Practical Approach (Borrebaeck, C., ed.), 1995, Oxford University Press, Oxford; J. Immunol. 149, 3914-3920 (1992)).

In addition, numerous publications have reported the use of phage display technology to produce and screen libraries of polypeptides for binding to a selected target. *See, e.g.,* Cwirla et al., Proc. Natl. Acad. Sci. USA 87, 6378-82, 1990; Devlin et al., Science 249,404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.,* in sandwich assays) may interfere with one another sterically, *etc.,* assay performance of an antibody may be a more important measure than absolute affinity and specificity of an antibody.

Those skilled in the art will recognize that many approaches can be taken in producing antibodies or binding fragments and screening and selecting for affinity and specificity for the various polypeptides, but these approaches do not change the scope of the invention.

### Examples

The following examples serve to illustrate the present invention. These examples are in no way intended to limit the scope of the invention.

### Example 1. Description of Patient Populations

### Normal

This population consisted of apparently healthy, normal subjects for use as "control" subjects in the development of new diagnostic tests. Inclusion criteria were (1) 18-65 years of age at the time of enrollment and (2) competent to give written informed consent. Exclusion criteria were (1) presence of any acute or chronic diseases (e.g. hypertension, diabetes, HIV), (2) active infection requiring antibiotics within 30 days of enrollment (e.g. urinary tract infection), (3) current evidence of alcohol or drug abuse, (4) history of malignancy within 5 years, except for non-melanoma skin cancer, (5) major trauma / surgery within the last 3 months, and (6) subjects who are considered by the investigator to be an unsuitable candidate for this population.

### Diastolic Dysfunction (DD)

Inclusion criteria for this population were patients > 18 years of age with intermediate and low-likelihood chest pain who were admitted to the hospital chest pain unit (CPU). To qualify for this study, patients were ruled out for myocardial infarction (MI) and had a negative stress echocardiogram performed as part of their CPU protocol. This stress echo served as an objective evidence of left ventricular (LV) ejection fraction, LV thickness and Doppler parameters of diastolic dysfunction. Exclusion criteria include initial evaluation/diagnosis that suggests epicardial coronary artery disease (CAD) or myocardial infarction, as well as known history of congestive heart failure (CHF) with left ventricular systolic dysfunction. Patients with LV dysfunction on their echocardiogram were also excluded.

### Pulmonary Embolism (PE)

This population included patients 18 years or older admitted with the diagnosis of PE. The criteria for the presence of PE are (1) a positive pulmonary angiogram, or (2) a high probability V/Q scan, or (3) a positive duplex study of the lower extremities and a high clinical suspicion for PE (defined as a clinical likelihood greater than 80% based on the assessment of the history, physical exam, arterial blood gas, chest film and electrocardiogram (ECG) by one of the clinical investigators) or (4) a high clinical suspicion with an abnormal echocardiogram in the absence of chronic pulmonary disease or (5) a positive helical CT angiogram. Patients presenting with left-sided heart failure, an acute coronary syndrome or renal insufficiency (creatinine > 1.5 mg/dl) were excluded.

### HF (Heart Failure)

This population included patients 18 years or older requiring treatment for HF. Patients with (1) current MI (myocardial infarction) or ACS (acute coronary syndrome) with ST deviation of 1 mm or greater, (2) renal failure requiring dialysis, (3) undergone hemodialysis within the last month, (4) enrollment (baseline) Triage® BNP concentration = 100 pg/ml or less, (5) enrolled in any other drug trial or receiving an experimental treatment for HF were excluded. This population also included patients 18 years or older who were hospitalized and treated for heart failure and then fully stabilized at discharge. Exclusion criteria for this sub-group included patients unable to give informed consent, history of severe pulmonary disease, chronic O₂ therapy for COPD, primary pulmonary hypertension, history of renal disease requiring dialysis, history of severe hepatic disease, patients using left ventricular assist devices (LVADs), stroke, MI, PTCA (percutaneous transluminal coronary artery angioplasty), CABG (coronary artery bypass graft) or unstable angina within the past 1 month, bi-ventricular pacemaker placement within last 1 month, history of severe aortic stenosis, history of severe mitral stenosis, history of constrictive pericarditis, patient has had a cardiac transplant, status 1 cardiac transplant candidates, ICD or DRG assignment of non-Q wave MI within last 1 month, and patient has cor pulmonale.

### RD (Renal Dysfunction)

This population consisted of hemodialysis patients. The patient population contained both females and males of 18-96 years of age. It is also referred to herein as "RF," or renal failure.

### Example 2. Assays

Blood samples were collected by trained personnel in standard blood collection tubes with EDTA as the anticoagulant. The plasma was separated from the cells by centrifugation, frozen, and stored at -20C or colder until analysis. The plasma was frozen within 1 hour. Assays were performed by standard immunoassay techniques using microfluidic devices essentially as described in Chapter 41, entitled "Near Patient Tests: Triage® Cardiac System," in The Immunoassay Handbook, 2nd ed., David Wild, ed., Nature Publishing Group, 2001.
BNP assay 1: A BNP sandwich immunoassay was formulated using two antibodies which bind within the BNP₇₉₋₁₀₈ sequence. In addition to BNP, this assay detects BNP₇₉₋₁₀₈, proBNP, and BNP₃₋₁₀₈.
BNP assay 2: A BNP sandwich immunoassay was formulated using two antibodies which binds within the BNP₇₉₋₁₀₈ sequence, one of which is specific for the loss of residues 77 and 78 from BNP. This antibody is selected by phage display, in which a mouse was immunized with the BNP₇₉₋₁₀₈ sequence, and the resulting antibodies were selected against BNP₇₉₋₁₀₈ in the presence of an excess of BNP. This excess BNP competes for binding to any antibodies that are not specific for the BNP₇₉₋₁₀₈ amino terminus. This assay does not detect BNP, proBNP, or BNP₃₋₁₀₈.
proBNP assay 1: A proBNP sandwich immunoassay was formulated using one antibody which binds within the BNP₇₉₋₁₀₈ sequence and one antibody which binds within the BNP₃₋₇₆ sequence. In addition to proBNP, this assay detects BNP₃₋₁₀₈.
proBNP assay 2: A proBNP sandwich immunoassay was formulated using one antibody which bind within the BNP₇₉₋₁₀₈ sequence, one of which is specific for the loss of residues 1 and 2 from proBNP. This antibody is selected by phage display, in which a mouse was immunized with the BNP₃₋₁₀₈ sequence, and the resulting antibodies were selected against BNP₃₋₁₀₈ in the presence of an excess of proBNP. This excess proBNP competes for binding to any antibodies that are not specific for the BNP₃₋₁₀₈ amino terminus. This assay does not detect BNP, proBNP, NT-proBNP, or BNP₃₋₇₆.
NTproBNP assay 1: An NTproBNP sandwich immunoassay was formulated using two antibodies which binds within the BNP₃₋₇₆ sequence. In addition to NTproBNP, this assay detects proBNP, BNP₃₋₁₀₈, and BNP₃₋₇₆.
NTproBNP assay 2: An NT-proBNP sandwich immunoassay was formulated using one antibody which binds within the BNP₃₋₇₆ sequence, one of which is specific for the loss of residues 1 and 2 from proBNP. This antibody is selected by phage display, in which a mouse was immunized with the BNP₃₋₁₀₈ sequence, and the resulting antibodies were selected against BNP₃₋₁₀₈ in the presence of an excess of proBNP. This excess proBNP competes for binding to any antibodies that are not specific for the BNP₃₋₁₀₈ amino terminus. This assay does not detect BNP, proBNP, BNP₃₋₁₀₈, or NT-proBNP.

### Example 3. Distinguishing the pulmonary embolism population from the renal dysfunction population

For simultaneous measurement of NT-proBNP and proBNP an antibody binding to the middle of the NT-proBNP sequence was conjugated to a detectable label (fluorescent energy transfer latex, or "FETL." Two antibodies, one specific for the loss of residues 1 and 2 from proBNP and one that binds to BNP, were immobilized on separate capture areas along the detection lane of microfluidic devices constructed essentially as described in Chapter 41, entitled "Near Patient Tests: Triage® Cardiac System," in The Immunoassay Handbook, 2nd ed., David Wild, ed., Nature Publishing Group, 2001. The resulting "NT-proBNP" assay can detect both BNP₃₋₇₆ and BNP₃₋₁₀₈, while the resulting "proBNP" assay can measure BNP₃₋₁₀₈ but not BNP₃₋₇₆.

The two immunoassays on the lane were simultaneously calibrated using purified recombinant proBNP diluted gravimetrically into human EDTA plasma. The resulting immunoassay pairs were more specific for proBNP expressed from mammalian cells than proBNP expressed from bacterial cells approximately by 4-5 times in mass concentration. Once a specimen was run, the resulting assay signals were translated into concentrations value-assigned to the proBNP calibrators. The concentration of NT-proBNP (BNP₃₋₇₆) concentration was separated out by subtracting the result of the proBNP assay from the result of the NT-proBNP assay.

For simultaneous measurement of BNP and proBNP, an antibody binding to BNP was conjugated to the FETL. Two antibodies, one that binds to BNP, and that binds to proBNP were immobilized on separate capture areas along the detection lane of microfluidic devices. The resulting "BNP" assay can detect BNP, proBNP, and BNP₃₋₁₀₈, while the "proBNP" assay can measure proBNP and BNP₃₋₁₀₈ but not BNP.

The immunoassays were calibrated using purified recombinant proBNP diluted gravimetrically into human EDTA plasma. The resulting immunoassay pairs were more specific for proBNP expressed from mammalian cells than proBNP expressed from bacterial cells approximately by 4-5 times in mass concentration. Once a specimen was run, the resulting assay signals were translated into concentrations value-assigned to the proBNP calibrators.

The concentration of NT-proBNP (BNP₃₋₇₆) was separated out by subtracting the result of the proBNP assay from the result of the NT-proBNP assay. The concentration of BNP was separated out from the BNP assay by subtracting the result from the proBNP assay from the result from the BNP assay. The "proBNP" assay on the second devices correlates well with the "proBNP" assay on the first devices (Spearman correlation R = 0.98 with p < 0.0001), while yielding a slightly larger result: [proBNP assay of BNP Panel] = 1.46 [proBNP assay of NT-proBNP Panel] -41.28 pg/ml by Passing-Bablok regression analysis (p < 0.01). The 95% confidence interval of the slope is 1.41 to 1.52. This difference in test result can be attributed to the difference in antibody specificity.

The following descriptive statistics were obtained using these assays. Measurement values are reported as pg/mL proBNP:

| **BNP Assay** | **Normal** | **DD** | **PE** | **HF** | **RD** |
|---|---|---|---|---|---|
| Count | 77 | 81 | 84 | 133 | 442 |
| 5th percentile | 0.00 | 0.00 | 0.00 | 457.94 | 87.11 |
| 25th percentile | 6.47 | 4.31 | 14.98 | 1610.88 | 473.33 |
| 50th percentile | 22.03 | 24.16 | 195.30 | 4184.45 | 2014.85 |
| 75th percentile | 89.59 | 147.55 | 876.71 | 8365.82 | 5394.15 |
| 95th percentile | 632.00 | 437.74 | 5342.40 | 16631.64 | 16018.50 |

| **proBNP Assay** | **Normal** | **DD** | **PE** | **HF** | **RF** |
|---|---|---|---|---|---|
| Count | 77 | 81 | 84 | 133 | 442 |
| 5th percentile | 0.00 | 0.00 | 0.00 | 255.46 | 108.28 |
| 25th percentile | 7.46 | 4.98 | 12.18 | 1001.28 | 404.32 |
| 50th percentile | 25.93 | 21.33 | 91.65 | 2287.90 | 1031.93 |
| 75th percentile | 57.23 | 67.08 | 525.52 | 4030.23 | 2724.97 |
| 95th percentile | 402.41 | 202.38 | 2946.52 | 9634.88 | 6348.33 |

| **NT-proBNP Assay** | **Normal** | **DD** | **PE** | **HF** | **RF** |
|---|---|---|---|---|---|
| Count | 76 | 81 | 78 | 73 | 182 |
| 5th percentile | 60.53 | 0.93 | 45.93 | 3794.41 | 5165.19 |
| 25th percentile | 181.21 | 98.59 | 264.77 | 12641.53 | 14388.59 |
| 50th percentile | 659.62 | 303.67 | 2195.31 | 27455.58 | 22253.77 |
| 75th percentile | 1660.44 | 1470.09 | 9500.87 | 38374.03 | 35639.36 |
| 95th percentile | 7570.75 | 2977.98 | 38241.53 | 48099.11 | 47336.80 |

Using this data, ROC analysis was performed to compare various groups (Figs. 1 and 2). SPSS 15.0 for Windows (SPSS, Chicago, IL) was used for this ROC analysis. The ratio of NT-proBNP to proBNP is superior to any other biomarkers in distinguishing HF from RD and PE from RD (ROC areas are close to 0.95), while conventional BNP and NT-proBNP assays, which also detect proBNP, poorly differentiate PE from RD and HF from RD respectively (ROC areas = ∼ 0.50).

The examples provided herein are representative of preferred embodiments, are exemplary, and are not intended as limitations on the scope of the invention. Modifications therein and other uses will occur to those skilled in the art.

## Claims

1. A method of assigning a diagnosis to a subject, comprising:
performing at least two immunoassays selected from the group consisting of an immunoassay configured to detect BNP₇₉₋₁₀₈; an immunoassay configured to detect BNP₃₋₇₆ but not to detect BNP; and an immunoassay configured to detect BNP₃₋₁₀₈ but not to detect BNP on one or more body fluid samples obtained from the subject;
deriving the concentration values from the results obtained from the immunoassays selected from the group consisting of a proBNP concentration substantially indicative of proBNP or BNP₃₋₁₀₈, and an NT-proBNP concentration substantially indicative of NT-proBNP or BNP₃₋₇₆;
determining a combined natriuretic peptide result that is a function of said proBNP concentration, and said NT-proBNP concentration; and
correlating the combined natriuretic peptide result to one diagnosis for the subject selected from the group consisting of the presence of renal dysfunction, the absence of renal dysfunction.

2. The method of claim 1, wherein said method comprises distinguishing between a diagnosis of renal dysfunction and a diagnosis of pulmonary embolism in said subject.

3. The method of claim 1, wherein the immunoassay configured to detect BNP₇₉₋₁₀₈ is configured as a sandwich immunoassay using one antibody that is specific for the loss of residues 77 and 78 from the BNP molecule, and a second antibody that binds to both BNP and BNP₇₉₋₁₀₈.

4. The method of claim 1, wherein the immunoassay configured to detect BNP₇₉₋₁₀₈ is configured as a sandwich immunoassay using two antibodies, each of which bind both BNP and BNP₇₉₋₁₀₈.

5. The method of claim 1, wherein the immunoassay configured to detect BNP₃₋₁₀₈ but not to detect BNP is configured as a sandwich immunoassay using one antibody that is specific for the loss of residues 1 and 2 from the proBNP molecule, and a second antibody that binds to both proBNP and BNP₃₋₁₀₈.

6. The method of claim 1, wherein the immunoassay configured to detect BNP₃₋₁₀₈ but not to detect BNP is configured as a sandwich immunoassay using two antibodies, each of which bind both proBNP and BNP₃₋₁₀₈.

7. The method of claim 1, wherein the immunoassay configured to detect BNP₃₋₇₆ but not to detect BNP is configured as a sandwich immunoassay using one antibody that is specific for the loss of residues 1 and 2 from the proBNP molecule, and a second antibody that binds to both NT-proBNP and BNP₃₋₇₆.

8. The method of claim 1, wherein the immunoassay configured to detect BNP₃₋₇₆ but not to detect BNP is configured as a sandwich immunoassay using two antibodies, each of which bind both NT-proBNP and BNP₃₋₇₆.

9. The method of claim 1, wherein the proBNP concentration is determined by computationally removing contributions of one or more of BNP, BNP₇₉₋₁₀₈, NT-proBNP and BNP₃₋₇₆ from a concentration determined from the immunoassay configured to detect BNP₃₋₁₀₈.

10. The method of claim 1, wherein the NT-proBNP concentration is determined by computationally removing contributions of one or both of proBNP and BNP₃₋₁₀₈ from a concentration determined from the immunoassay configured to detect BNP₃₋₇₆, wherein an NT-proBNP/proBNP ratio is calculated by dividing said NT-proBNP concentration by a proBNP concentration to provide said combined natriuretic peptide result.

11. The method of claim 1, wherein the combined natriuretic peptide result is a function of: a proBNP concentration and an NT-proBNP concentration, and wherein the function is selected from the group consisting of a proBNP/NT-proBNP ratio, and an NT-proBNP/proBNP ratio.

12. The method of claim 2, wherein the combined natriuretic peptide result is a ratio of NT-proBNP to proBNP calculated using a proBNP concentration substantially indicative of proBNP or BNP₃₋₁₀₈, and an NT-proBNP concentration substantially indicative of NT-proBNP or BNP₃₋₇₆; and wherein said correlating step comprises comparing the combined natriuretic peptide result to a threshold value selected to distinguish heart failure from renal dysfunction.

13. The method of claim 2, wherein the combined natriuretic peptide result is a ratio of NT-proBNP to proBNP calculated using a proBNP concentration substantially indicative of proBNP or BNP₃₋₁₀₈, and an NT-proBNP concentration substantially indicative of NT-proBNP or BNP₃₋₇₆; and
wherein said correlating step comprises comparing the combined natriuretic peptide result to a threshold value selected to distinguish pulmonary embolism from renal dysfunction.

14. The method of claim 2, wherein the combined natriuretic peptide result is a ratio of NT-proBNP to proBNP calculated using a proBNP concentration substantially indicative of proBNP or BNP₃₋₁₀₈, and an NT-proBNP concentration substantially indicative of NT-proBNP or BNP₃₋₇₆; and
wherein said correlating step comprises comparing the combined natriuretic peptide result to a threshold value selected to distinguish both pulmonary embolism and heart failure from renal dysfunction.

## Patentansprüche

1. Verfahren zum Zuweisen einer Diagnose zu einem Subjekt, umfassend:
Durchführen von mindestens zwei Immunassays, ausgewählt aus der Gruppe bestehend aus einem Immunassay, der dazu konfiguriert ist, BNP₇₉₋₁₀₈ nachzuweisen, einem Immunassay, der dazu konfiguriert ist, BNP₃₋₇₆ nachzuweisen aber nicht BNP nachzuweisen, und einem Immunassay, der dazu konfiguriert ist, BNP₃₋₁₀₈ nachzuweisen aber nicht BNP nachzuweisen, mit einer oder mehreren Körperflüssigkeitsproben, die aus dem Subjekt erhalten wurden;
Ableiten der Konzentrationswerte aus den Ergebnissen, die von den Immunassays erhalten wurden, ausgewählt aus der Gruppe bestehend aus einer proBNP-Konzentration, die im wesentlichen proBNP oder BNP₃₋₁₀₈ anzeigt, und einer NT-proBNP-Konzentration, die im wesentlichen NT-proBNP oder BNP₃₋₇₆ anzeigt;
Bestimmen eines kombinierten Ergebnisses für natriuretisches Peptid, das eine Funktion der genannten proBNP-Konzentration und der genannten NT-proBNP-Konzentration ist; und
Korrelieren des kombinierten Ergebnisses für natriuretisches Peptid mit einer Diagnose für das Subjekt, die aus der Gruppe bestehend aus der Gegenwart von Nierenfunktionsstörung, der Abwesenheit von NierenFunktionsstörung, ausgewählt ist.

2. Verfahren gemäß Anspruch 1, wobei das Verfahren das Unterscheiden zwischen einer Diagnose von Nierenfunktionsstörung und einer Diagnose von Lungenembolie in dem Subjekt umfasst.

3. Verfahren gemäß Anspruch 1, wobei der Immunassay, der dazu konfiguriert ist, BNP₇₉₋₁₀₈ nachzuweisen, als ein Sandwich-Immunassay konfiguriert ist, welcher einen Antikörper verwendet, der für den Verlust der Reste 77 und 78 aus dem BNP-Molekül spezifisch ist, und einen zweiten Antikörper verwendet, der sowohl an BNP als auch an BNP₇₉₋₁₀₈ bindet.

4. Verfahren gemäß Anspruch 1, wobei der Immunassay, der dazu konfiguriert ist, BNP₇₉₋₁₀₈ nachzuweisen, als ein Sandwich-Immunassay konfiguriert ist, welcher zwei Antikörper verwendet, von denen jeder sowohl BNP als auch BNP₇₉₋₁₀₈ bindet.

5. Verfahren gemäß Anspruch 1, wobei der Immunassay, der dazu konfiguriert ist, BNP₃₋₁₀₈ nachzuweisen aber nicht BNP nachzuweisen, als ein Sandwich-Immunassay konfiguriert ist, welcher einen Antikörper verwendet, der für den Verlust der Reste 1 und 2 aus dem proBNP-Molekül spezifisch ist, und einen zweiten Antikörper verwendet, der sowohl an proBNP als auch an BNP₃₋₁₀₈ bindet.

6. Verfahren gemäß Anspruch 1, wobei der Immunassay, der dazu konfiguriert ist, BNP3-108 nachzuweisen aber nicht BNP nachzuweisen, als ein Sandwich-Immunassay konfiguriert ist, welcher zwei Antikörper verwendet, von denen jeder sowohl proBNP als auch BNP3-108 bindet.

7. Verfahren gemäß Anspruch 1, wobei der Immunassay, der dazu konfiguriert ist, BNP₃₋₇₆ nachzuweisen aber nicht BNP nachzuweisen, als ein Sandwich-Immunassay konfiguriert ist, welcher einen Antikörper verwendet, der für den Verlust der Reste 1 und 2 aus dem proBNP-Molekül spezifisch ist, und einen zweiten Antikörper verwendet, der sowohl NT-proBNP als auch BNP₃₋₇₆ bindet.

8. Verfahren gemäß Anspruch 1, wobei der Immunassay, der dazu konfiguriert ist, BNP₃₋₇₆ nachzuweisen aber nicht BNP nachzuweisen, als ein Sandwich-Immunassay konfiguriert ist, welcher zwei Antikörper verwendet, von denen jeder sowohl NP-proBNP als auch BNP₃₋₇₆ bindet.

9. Verfahren gemäß Anspruch 1, wobei die proBNP-Konzentration bestimmt wird, indem rechnerisch Beiträge von einem oder mehreren von BNP, BNP₇₉₋₁₀₈, NT-proBNP und BNP₃₋₇₆ aus einer Konzentration entfernt werden, die aus dem Immunassay bestimmt wurde, welcher dazu konfiguriert ist, BNP₃₋₁₀₈ nachzuweisen.

10. Verfahren gemäß Anspruch 1, wobei die NT-proBNP-Konzentration bestimmt wird, indem rechnerisch Beiträge von einem oder zwei von proBNP und BNP₃₋₁₀₈ aus einer Konzentration entfernt werden, die aus dem Immunassay bestimmt wurde, welcher dazu konfiguriert ist, BNP₃₋₇₆ nachzuweisen, wobei ein NT-proBNP/proBNP-Verhältnis berechnet wird, indem die NT-proBNP-Konzentration durch eine proBNP-Konzentration geteilt wird, um das kombinierte Ergebnis für natriuretisches Peptid bereitzustellen.

11. Verfahren gemäß Anspruch 1, wobei das kombinierte Ergebnis für natriuretisches Peptid eine Funktion ist von: einer proBNP-Konzentration und einer NT-proBNP-Konzentration, und wobei die Funktion ausgewählt ist aus der Gruppe bestehend aus einem proBNP/NT-proBNP-Verhältnis und einem NT-proBNP/proBNP-Verhältnis.

12. Verfahren gemäß Anspruch 2, wobei das kombinierte Ergebnis für natriuretisches Peptid ein Verhältnis von NT-proBNP zu proBNP ist, berechnet mittels einer proBNP-Konzentration, welche im wesentlichen proBNP oder BNP₃₋₁₀₈ anzeigt, und einer NT-proBNP-Konzentration, welche im wesentlichen NT-pro-BNP oder BNP₃₋₇₆ anzeigt, und wobei der Schritt des Korrelierens das Vergleichen des kombinierten Ergebnisses für natriuretisches Peptid mit einem Schwellenwert umfasst, welcher gewählt wird, um Herzinsuffizienz von Nierenfunktionsstörung zu unterscheiden.

13. Verfahren gemäß Anspruch 2, wobei das kombinierte Ergebnis für natriuretisches Peptid ein Verhältnis von NT-proBNP zu proBNP ist, berechnet mittels einer proBNP-Konzentration, welche im wesentlichen proBNP oder BNP₃₋₁₀₈ anzeigt, und einer NT-proBNP-Konzentration, welche im wesentlichen NT-proBNP oder BNP₃₋₇₆ anzeigt;
und
wobei der Schritt des Korrelierens das Vergleichen des kombinierten Ergebnisses für natriuretisches Peptid mit einem Schwellenwert umfasst, der gewählt ist, um Lungenembolie von Nierenfunktionsstörung zu unterscheiden.

14. Verfahren gemäß Anspruch 2, wobei das kombinierte Ergebnis für natriuretisches Peptid ein Verhältnis von NT-proBNP zu proBNP ist, berechnet mittels einer proBNP-Konzentration, welche im wesentlichen proBNP oder BNP₃₋₁₀₈ anzeigt, und einer NT-proBNP-Konzentration, welche im wesentlichen NT-proBNP oder BNP₃₋₇₆ anzeigt;
und
wobei der Schritt des Korrelierens das Vergleichen des kombinierten Ergebnisses für natriuretisches Peptid mit einem Schwellenwert umfasst, der gewählt ist, um sowohl Lungenembolie als auch Herzinsuffizienz von Nierenfunktionsstörung zu unterscheiden.

## Revendications

1. Procédé d'attribution d'un diagnostic à un sujet, comprenant le fait :
d'effectuer au moins deux immuno-essais choisis dans le groupe constitué d'un immuno-essai configuré pour détecter le BNP₇₉₋₁₀₈ ; un immuno-essai configuré pour détecter le BNP₃₋₇₆ mais pas pour détecter le BNP ; et un immuno-essai configuré pour détecter le BNP₃₋₁₀₈ mais pas pour détecter le BNP sur un ou plusieurs échantillon(s) de fluide corporel obtenus à partir du sujet ;
de dériver les valeurs de concentration provenant des résultats obtenus à partir des immuno-essais choisies dans le groupe constitué d'une concentration de proBNP indiquant substantiellement le proBNP ou le BNP₃₋₁₀₈, et d'une concentration de NT-proBNP indiquant substantiellement le NT-proBNP ou le BNP₃₋₇₆ ;
de déterminer un résultat de peptide natriurétique combiné qui est une fonction de ladite concentration de proBNP, et de ladite concentration de NT-proBNP ; et
de corréler le résultat de peptide natriurétique combiné à un diagnostic pour le sujet choisi dans le groupe constitué de la présence de dysfonctionnement rénal, l'absence de dysfonctionnement rénal.

2. Procédé de la revendication 1, dans lequel ledit procédé comprend le fait de distinguer entre un diagnostic de dysfonctionnement rénal et un diagnostic d'embolie pulmonaire chez ledit sujet.

3. Procédé de la revendication 1, dans lequel l'immuno-essai configuré pour détecter le BNP₇₉₋₁₀₈ est configuré en tant qu'immuno-essai en sandwich utilisant un anticorps qui est spécifique pour la perte de résidus 77 et 78 de la molécule BNP, et un deuxième anticorps qui se lie à la fois au BNP et au BNP₇₉₋₁₀₈.

4. Procédé de la revendication 1, dans lequel l'immuno-essai configuré pour détecter le BNP₇₉₋₁₀₈ est configuré en tant qu'immuno-essai en sandwich utilisant deux anticorps, dont chacun se lie à la fois au BNP et au BNP₇₉₋₁₁₈.

5. Procédé de la revendication 1, dans lequel l'immuno-essai configuré pour détecter le BNP₃₋₁₀₈ mais pas pour détecter le BNP est configuré en tant qu'immuno-essai en sandwich utilisant un anticorps qui est spécifique pour la perte de résidus 1 et 2 de la molécule proBNP, et un deuxième anticorps qui se lie à la fois au proBNP et au BNP₃₋₁₀₈.

6. Procédé de la revendication 1, dans lequel l'immuno-essai configuré pour détecter le BNP₃₋₁₀₈ mais pas pour détecter le BNP est configuré en tant qu'immuno-essai en sandwich utilisant deux anticorps, dont chacun se lie à la fois au proBNP et au BNP₃₋₁₀₈.

7. Procédé de la revendication 1, dans lequel l'immuno-essai configuré pour détecter le BNP₃₋₇₆ mais pas pour détecter le BNP est configuré en tant qu'immuno-essai en sandwich utilisant un anticorps qui est spécifique pour la perte de résidus 1 et 2 de la molécule proBNP, et un deuxième anticorps qui se lie à la fois au NT-proBNP et au BNP₃₋₇₆.

8. Procédé de la revendication 1, dans lequel l'immuno-essai configuré pour détecter le BNP₃₋₇₆ mais pas pour détecter le BNP est configuré en tant qu'immuno-essai en sandwich utilisant deux anticorps, dont chacun se lie à la fois au NT-proBNP et au BNP₃₋₇₆.

9. Procédé de la revendication 1, dans lequel la concentration de proBNP est déterminée en éliminant par calcul des contributions d'un ou de plusieurs élément(s) parmi le BNP, BNP₇₉₋₁₀₈, le NT-proBNP et le BNP₃₋₇₆ d'une concentration déterminée à partir de l'immuno-essai configuré pour détecter le BNP₃₋₁₀₈,

10. Procédé de la revendication 1, dans lequel la concentration de NT-proBNP est déterminée en éliminant par calcul des contributions du proBNP et/ou de BNP₃₋₁₀₈ d'une concentration déterminée à partir de l'immuno-essai configuré pour détecter le BNP₃₋₇₆, où un rapport NT-proBNP/proBNP est calculé en divisant ladite concentration de NT-proBNP par une concentration de proBNP pour fournir ledit résultat de peptide natriurétique combiné.

11. Procédé de la revendication 1, dans lequel le résultat de peptide natriurétique combiné est une fonction : d'une concentration de proBNP et d'une concentration de NT-proBNP, et où la fonction est choisie dans le groupe constitué d'un rapport proBNP/NT-proBNP, et d'un rapport NT-proBNP/proBNP.

12. Procédé de la revendication 2, dans lequel le résultat de peptide natriurétique combiné est un rapport de NT-proBNP sur proBNP calculé en utilisant une concentration de proBNP indiquant substantiellement le proBNP ou le BNP₃₋₁₀₈, et une concentration de NT-proBNP indiquant substantiellement le NT-proBNP ou le BNP₃₋₇₆ ; et où ladite étape de corrélation comprend le fait de comparer le résultat de peptide natriurétique combiné à une valeur seuil choisie pour distinguer une insuffisance cardiaque d'un dysfonctionnement rénal.

13. Procédé de la revendication 2, dans lequel le résultat de peptide natriurétique combiné est un rapport de NT-proBNP sur le proBNP calculé en utilisant une concentration de proBNP indiquant substantiellement le proBNP ou le BNP₃₋₁₀₈, et une concentration de NT-proBNP indiquant substantiellement le NT-proBNP ou le BNP₃₋₇₆ ; et
dans lequel ladite étape de corrélation comprend le fait de comparer le résultat de peptide natriurétique combiné à une valeur seuil choisie pour distinguer une embolie pulmonaire d'un dysfonctionnement rénal.

14. Procédé de la revendication 2, dans lequel le résultat de peptide natriurétique combiné est un rapport de NT-proBNP sur le proBNP calculé en utilisant une concentration de proBNP indiquant substantiellement le proBNP ou le BNP₃₋₁₀₈, et une concentration de NT-proBNP indiquant substantiellement le NT-proBNP ou le BNP₃₋₇₆ ; et
dans lequel ladite étape de corrélation comprend le fait de comparer le résultat de peptide natriurétique combiné à une valeur seuil choisie pour distinguer à la fois une embolie pulmonaire et une insuffisance cardiaque d'un dysfonctionnement rénal.
